# EUROPÄISCHE PATENTANMELDUNG

(11) **EP 1 683 789 A1**
(43) Veröffentlichungstag der Anmeldung: **26.07.2006**
(21) Anmeldenummer: 06009626.0
(22) Anmeldetag: 20.02.1997
(51) Int. Cl.: C07D 251/16, C07D 251/22, C07D 251/46, C07D 239/42, C07D 239/46, A01N 47/36

(54) **Substituierte Arylsulfonyl(thio)harnstoffe als Herbizide**

(30) Priorität: 05.03.1996 DE 19608445
(62) Teilanmeldung aus: 97906098.5
(71) Anmelder: Bayer CropScience AG, 40789 Monheim am Rhein (DE)
(72) Erfinder: Gesing, Ernst R.F., 4699 Erkrath-Hochdahl (DE); Kirsten, Rolf, 40789 Monheim (DE); Kluth, Joachim, 40764 Langenfeld (DE); Müller, Klaus-Helmut, 40593 Düsseldorf (DE); Drewes, Mark Wilhelm, 40789 Monheim (DE); Jansen, Johannes R., 40789 Monheim (DE); Philipp, Ulrich, Lees Summit MO 64064 (US); Riebel, Hans-Jochem, 56242 Seiters (DE); Schallner, Otto, 40789 Monheim (DE); Dollinger, Markus, 51381 Leverkusen (DE); Santel, Hans-Joachim, 51371 Leverkusen (DE)
(74) Vertreter: Schwenk, Norbert

(57) **Zusammenfassung**

Die Erfindung betrifft neue substituierte Arylsulfonyl(thio)harnstoffe 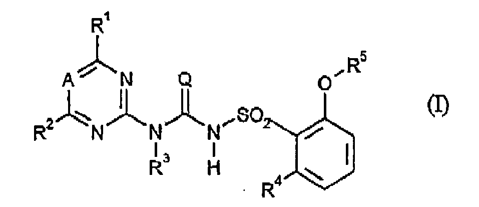 in welcher
- A: für Stickstoff oder eine CH-Gruppierung steht,
- Q: für Sauerstoff oder Schwefel steht,
- R¹: für Wasserstoff, Halogen oder jeweils gegebenenfalls substituiertes Alkyl, Alkoxy, Alkylthio, Alkylamino, Dialkylamino, Cycloalkyl, Cycloalkyloxy oder Heterocyclyloxy steht,
- R²: für Wasserstoff, Halogen oder jeweils gegebenenfalls substituiertes Alkyl, Alkoxy, Alkylthio, Alkylamino, Dialkylamino, Cycloalkyl, Cycloalkyloxy oder Heterocyclyloxy steht,
- R³: für Wasserstoff oder gegebenenfalls substituiertes Alkyl steht,
- R⁴: für jeweils gegebenenfalls substituiertes Alkyl, Alkoxy, Alkenyl, Alkinyl, Alkenyloxy, Alkinyloxy, Cycloalkyl, Cycloalkyloxy oder Cycloalkylalkyl steht, und

- R⁵: für Wasserstoff, Formyl oder jeweils gegebenenfalls substituiertes Alkyl, Alkylcarbonyl, Alkoxycarbonyl, Alkylsulfonyl, Alkenyl, Alkinyl, Cycloalkyl, Cycloalkylcarbonyl, Cycloalkylsulfonyl oder Heterocyclyl steht,
sowie Salze von Verbindungen der Formel (I),
wobei die Verbindung N-(4,6-Dimethyl-pyrimidin-2-yl)-N'-[2-(1,1,2,2-tetrafluorethoxy)-6-methyl-phenylsulfonyl]-harnstoff ausgenommen ist,
Verfahren zur Herstellung der neuen Verbindungen und ihre Verwendung als Herbizide.

## Beschreibung

Die Erfindung betrifft neue substituierte Arylsulfonyl(thio)harnstoffe, Verfahren zu ihrer Herstellung und ihre Verwendung als Herbizide.

Es ist bereits bekannt, daß bestimmte substituierte Sulfonylharnstoffe herbizide Eigenschaften aufweisen (vgl. DE 2715786, EP 1514, EP 23422). Die herbizide Wirksamkeit und die Verträglichkeit gegenüber Kulturpflanzen dieser Verbindungen sind jedoch nicht in allen Belangen zufriedenstellend.

Es wurden nun die neuen substituierten Arylsulfonyl(thio)harnstoffe der allgemeinen Formel (I) 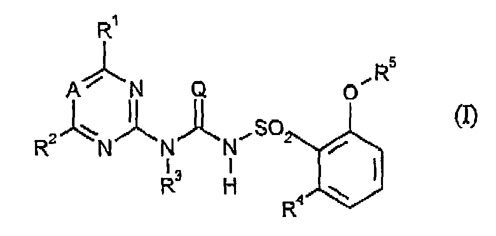 in welcher
- A: für Stickstoff oder eine CH-Gruppierung steht,
- Q: für Sauerstoff oder Schwefel steht,
- R¹: für Wasserstoff, Halogen oder jeweils gegebenenfalls substituiertes Alkyl, Alkoxy, Alkylthio, Alkylamino, Dialkylamino, Cycloalkyl, Cycloalkyloxy oder Heterocyclyloxy steht,
- R²: für Wasserstoff, Halogen oder jeweils gegebenenfalls substituiertes Alkyl, Alkoxy, Alkylthio, Alkylamino, Dialkylamino, Cycloalkyl, Cycloalkyloxy oder Heterocyclyloxy steht,
- R³: für Wasserstoff oder gegebenenfalls substituiertes Alkyl steht,

- R⁴: für jeweils gegebenenfalls substituiertes Alkyl, Alkoxy, Alkenyl, Alkinyl, Alkenyloxy, Alkinyloxy, Cycloalkyl, Cycloalkyloxy oder cycloakylakyl steht, und
- R⁵: für Wasserstoff, Formyl oder jeweils gegebenenfalls substituiertes Alkyl, Alkylcarbonyl, Alkoxycarbonyl, Alkylsulfonyl, Alkenyl, Alkinyl, Cycloalkyl, Cycloalkylcarbonyl, Cycloalkylsulfonyl oder Heterocyclyl steht,
sowie Salze von Verbindungen der Formel (I) gefunden,
wobei die vorbekannte Verbindung N-(4,6-Dimethyl-pyrimidin-2-yl)-N'-[2-(1,1,2,2-tetrafluor-ethoxy)-6-methyl-phenylsulfonyl]-harnstoff (vgl. EP 23422) durch Disclaimer ausgenommen ist.

Man erhält die neuen substituierten Arylsulfonyl(thio)harstoffe der allgemeinen Formel (I), wenn man
(a) Aminoazine der allgemeinen Formel (II) 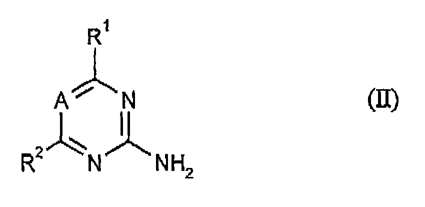 in welcher
   A, R¹ und R² die oben angegebene Bedeutung haben,
   mit Arylsulfonyliso(thio)cyanaten der allgemeinen Formel (III) 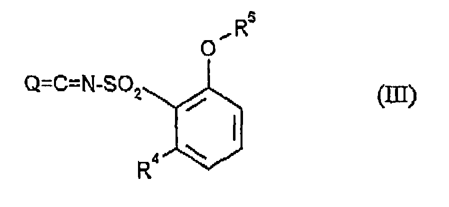 in welcher
   Q, R⁴ und R⁵ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(b) substituierte Aminoazine der allgemeinen Formel (IV) 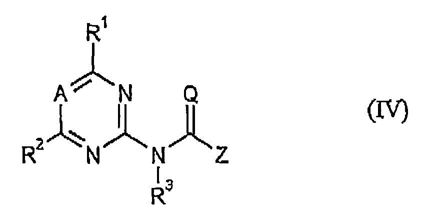 in welcher
   A, Q, R¹ und R² die oben angegebene Bedeutung haben,
   Z für Halogen, Alkoxy oder Aryloxy steht und
   R³ die oben angegebene Bedeutung hat oder für die Gruppierung -C(Q)-Z steht,
   mit Arensulfonamiden der allgemeinen Formel (V) 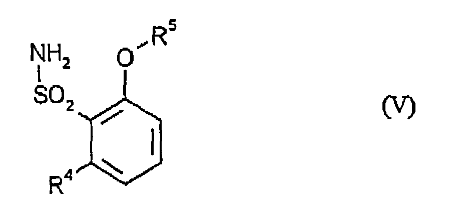 in welcher
   R⁴ und R⁵ die oben angegebene Bedeutung haben,
   gegebenenfalls in Gegenwart eines Reaktionshilfsmittels und gegebenenfalls in Gegenwart eines Verdünnungsmittels umsetzt,
   oder wenn man
(c) Aminoazine der allgemeinen Formel (II) 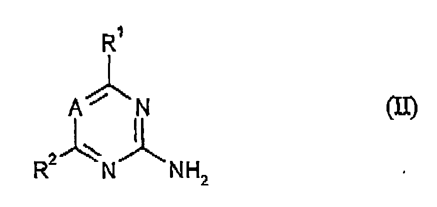 in welcher
   A, R¹ und R² die oben angegebene Bedeutung haben,
   mit substituierten Arensulfonamiden der allgemeinen Formel (VI) 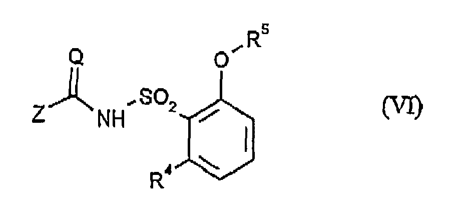 in welcher
   Q, R⁴ und R⁵ die oben angegebene Bedeutung haben und
   Z für Halogen, Alkoxy oder Aryloxy steht,
   gegebenenfalls in Gegenwart eines Reaktionshilfsmittels und gegebenenfalls in Gegenwart eines Verdünnungsmittels umsetzt,
   und gegebenenfalls die nach den Verfahren (a), (b) und (c) erhaltenen Verbindungen der Formel (I) nach üblichen Methoden in Salze überführt.

Die neuen substituierten Arylsulfonyl(thio)harnstoffe der allgemeinen Formel (I) zeichnen sich durch starke herbizide Wirksamkeit aus.

Gegenstand der Erfindung sind vorzugsweise Verbindungen der Formel (I), in welcher
- A: für Stickstoff oder eine CH-Gruppierung steht,
- Q: für Sauerstoff oder Schwefel steht,
- R¹: für Wasserstoff, Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Oxetanyloxy, Furyloxy oder Tetrahydrofuryloxy steht,
- R²: für Wasserstoff oder Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Akyl oder C₁-C₄-Alkoxy substituiertes Oxetanyloxy, Furyloxy oder Tetrahydrofuryloxy steht,
- R³: für Wasserstoff oder gegebenenfalls durch C₁-C₄-Alkoxy, C₁-C₄-Alkylcarbonyl oder C₁-C₄-Alkoxy-carbonyl substituiertes Alkyl mit 1 bis 4 Kohlenstoffatomen steht,
- R⁴: für jeweils gegebenenfalls durch Cyano, Halogen oder C₁-C₄-Alkoxy substituiertes Alkyl oder Alkoxy mit jeweils 1 bis 6 Kohlenstoffatomen in den Alkylgruppen, für jeweils gegebenenfalls durch Halogen substituiertes Alkenyl, Alkinyl, Alkenyloxy oder Alkinyloxy mit jeweils 2 bis 6 Kohlenstoffatomen in den Alkenyl- oder Alkinyl-gruppen, oder für jeweils gegebenenfalls durch Cyano, Halogen oder C₁-C₄-Alkyl substituiertes Cycloalkyl, Cycloalkyloxy oder Cycloalkylalkyl mit jeweils 3 bis 6 Kohlenstoff atomen in den Cycloalkylgruppen und gegebenenfalls 1 bis 4 Kohlenstoffatomen im Alkylteil steht,
- R⁵: für Wasserstoff, Formyl, für jeweils gegebenenfalls durch Cyano, Halogen oder C₁-C₄-Alkoxy substituiertes Alkyl, Alkylcarbonyl, Alkoxycarbonyl oder Alkylsulfonyl mit jeweils 1 bis 6 Kohlenstoffatomen in den Alkylgruppen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl, Cycloalkylcarbonyl oder Cycloalkylsulfonyl mit jeweils 3 bis 6 Kohlenstoffatomen in den Cycloalkylgruppen, oder für jeweils gegebenenfalls durch Cyano, Halogen, C₁-C₄-Alkyl oder C₁-C₄-Alkoxy substituiertes Oxetanyl, Furyl oder Tetrahydrofuryl steht,
wobei die vorbekannte Verbindung N-(4,6-Dimethyl-pyrimidin-2-yl)-N'-[2-(1,1,2,2-tetrafluor-ethoxy)-6-methyl-phenylsulfonyl]-harnstoff (vgl. EP 23422) durch Disclaimer ausgenommen ist.

Gegenstand der Erfindung sind weiter vorzugsweise Natrium-, Kalium-, Magnesium-, Calcium-, Ammonium-, C₁-C₄-Alkyl-ammonium-, Di-(C₁-C₄-alkyl)-ammonium-, Tri-(C₁-C₄-alkyl)-ammonium-, Tetra-(C₁-C₄-alkyl)-ammonium-, Tri-(C₁-C₄-alkyl)-sulfonium-, C₅- oder C₆-Cycloalkyl-ammonium- und Di-(C₁-C₂-alkyl)-benzyl-ammomium-Salze von Verbindungen der Formel (I), in welcher A, Q, R¹, R², R³, R⁴ und R⁵ die oben vorzugsweise angegebenen Bedeutungen haben.

Gegenstand der Erfindung sind insbesondere Verbindungen der Formel (I), in welcher
- A: für Stickstoff oder eine CH-Gruppierung steht,
- Q: für Sauerstoff oder Schwefel steht,
- R¹: für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,

- R²: für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-propoxy, Methylthio, Ethylthio, n- oder i-Propylthio, Methylamino, Ethylamino, n- oder i-Propylamino, Dimethylamino oder Diethylamino steht,
- R³: für Wasserstoff oder gegebenenfalls durch Methoxy, Ethoxy, n- oder i-Propoxy, Acetyl, Propionyl, n- oder i-Butyroyl, Methoxycarbonyl, Ethoxycarbonyl, n- oder i-Propoxycarbonyl substituiertes Methyl oder Ethyl steht,
- R⁴: für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-kropoxy, n-, i-, s- oder t-Butoxy, für jeweils gegebenenfalls durch Fluor, Chlor oder Brom substituiertes Propenyl, Butenyl, Propinyl, Butinyl, Propenyloxy, Butenyloxy, Propinyloxy oder Butinyloxy, oder für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl oder Cyclohexylmethyl steht,
- R⁵: für Wasserstoff, Formyl, für jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Acetyl, Propionyl, n- oder i-Butyroyl, Methoxycarbonyl, Ethoxycarbonyl, n- oder i-Propoxycarbonyl, Methylsulfonyl, Ethylsulfonyl, n- oder i-Propylsulfonyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropylsulfonyl, Cyclobutylsulfonyl, Cyclopentylsulfonyl oder Cyclohexylsulfonyl steht,
wobei die vorbekannte Verbindung N-(4,6-Dimethyl-pyrimidin-2-yl)-N'-[2-(1,1,2,2-tetrafluor-ethoxy)-6-methyl-phenylsulfonyl]-harnstoff (vgl. EP 23422) durch Disclaimer ausgenommen ist.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte, Diese Restedefinitionen können untereinander, also auch zwischen den angegebenen Bereichen bevorzugter Verbindungen, beliebig kombiniert werden.

Beispiele für die erfindungsgemäßen Verbindungen der Formel (I) sind in den nachstehenden Gruppen aufgeführt.

### Gruppe 1

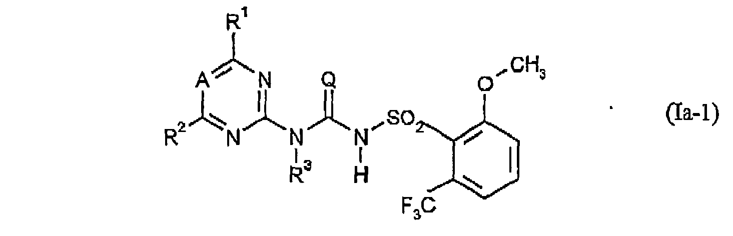

A, Q, R¹ R² und R³ haben darin beispielhaft die nachstehend aufgeführte. Bedeutung:

| A | Q | R¹ | R² | R³ |
|---|---|---|---|---|
| CH | O | OCH₃ | OCH₃ | H |
| CH | O | CH₃ | OCH₃ | H |
| CH | O | CH₃ | CH₃ | H |
| CH | O | Cl | OCH₃ | H |
| CH | O | H | CH₃ | H |
| N | O | CH₃ | OCH₃ | CH₃ |
| N | O | OCH₃ | OCH₃ | CH₃ |
| N | O | CH₃ | OCH₃ | H |
| N | O | OCH₃ | OCH₃ | H |
| N | O | CH₃ | CH₃ | H |
| N | O | OCHF₂ | N(CH₃)₂ | H |
| N | O | CH₃ | SCH₃ | H |
| N | O | C₂H₅ | OCH₃ | H |
| N | O | CH₃ | OC₂H₅ | H |
| N | O | H | OCH₃ | H |
| N | O | OCH₃ | 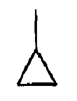 | H |
| N | O | CH₃ | N(CH₃)₂ | H |
| CH | O | OCH₃ | 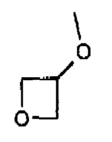 | H |
| CH | O | 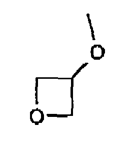 | 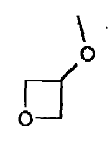 | H |
| CH | O | CH₃ | 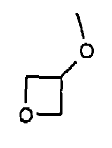 | H |
| CH | O | Cl | 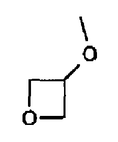 | H |
| N | O | 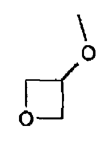 | 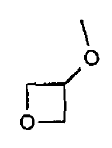 | H |
| N | O | CH₃ | 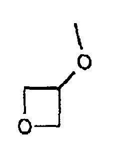 | H |
| N | O | H | 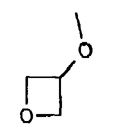 | H |
| N | S | CH₃ | OCH₃ | H |

### Gruppe 2

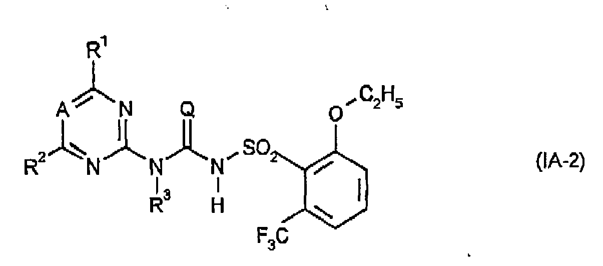

A, Q, R¹, R² und R³ haben hierbei beispielhaft die oben in Gruppe 1 angegebene Bedeutung.

### Gruppe 3

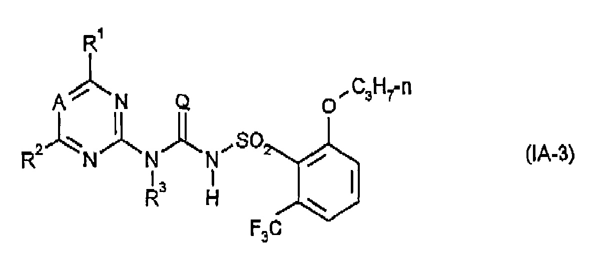

A, Q, R¹, R² und R³ haben hierbei beispielhaft die oben in Gruppe 1 angegebene Bedeutung.

### Gruppe 4

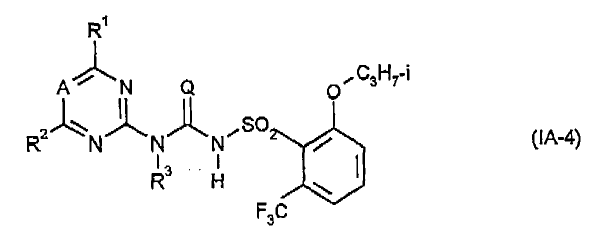

A, Q, R¹, R² und R³ haben hierbei beispielhaft die oben in Gruppe 1 angegebene Bedeutung.

### Gruppe 5

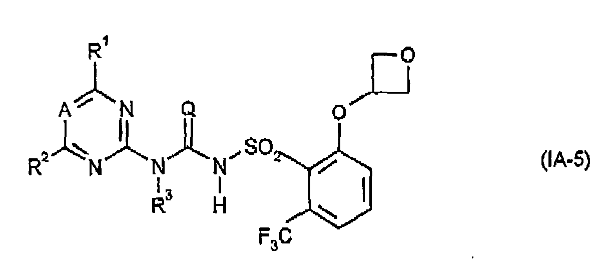

A, Q, R¹, R² und R³ haben hierbei beispielhaft die oben in Gruppe 1 angegebene Bedeutung.

### Gruppe 6

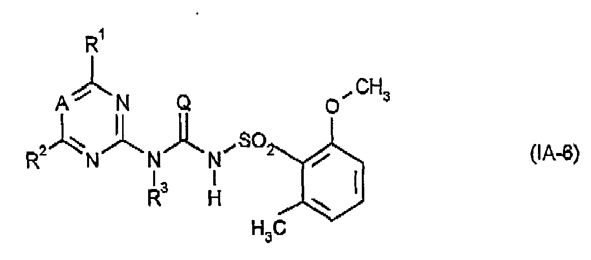

A, Q, R¹, R² und R³ haben hierbei beispielhaft die oben in Gruppe 1 angegebene Bedeutung.

### Gruppe 7

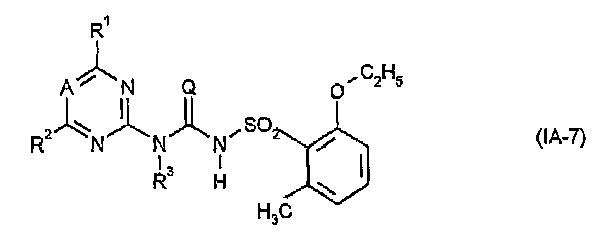

A, Q, R¹, R² und R³ haben hierbei beispielhaft die oben in Gruppe 1 angegebene Bedeutung.

### Gruppe 8

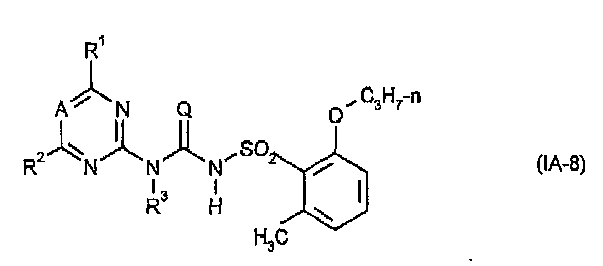

A, Q, R¹, R² und R³ haben hierbei beispielhaft die oben in Gruppe 1 angegebene Bedeutung.

### Gruppe 9

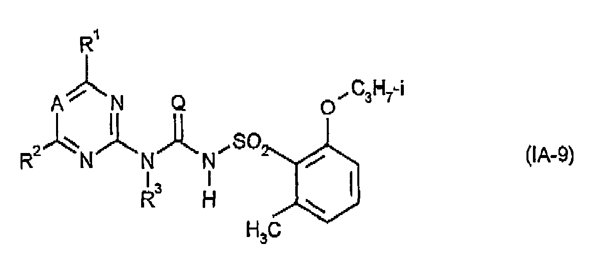

A, Q, R¹, R² und R³ haben hierbei beispielhaft die oben in Gruppe 1 angegebene Bedeutung.

### Gruppe 10

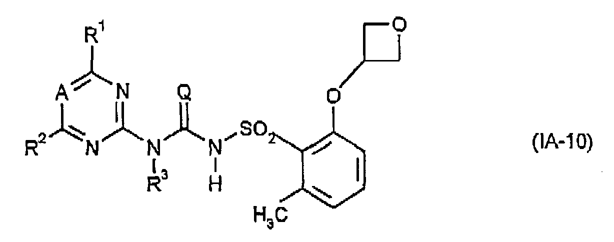

A, Q, R¹, R² und R³ haben hierbei beispielhaft die oben in, Gruppe 1 angegebene Bedeutung.

### Gruppe 11

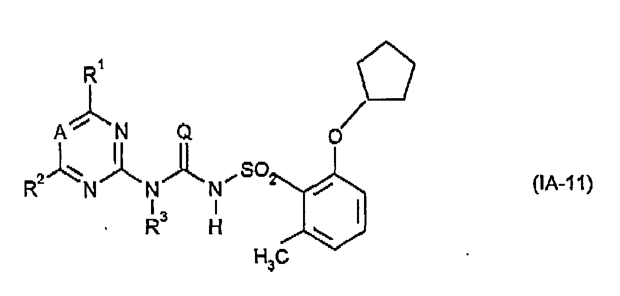

A, Q, R¹, R² und R³ haben hierbei beispielhaft die oben in Gruppe 1 angegebene Bedeutung.

### Gruppe 12

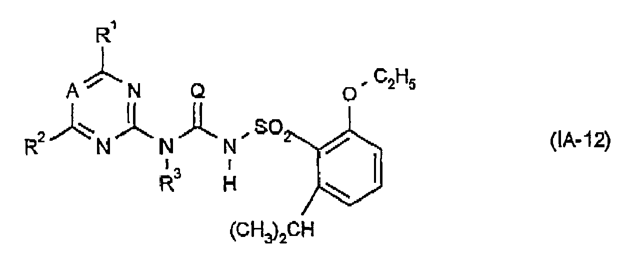

A, Q, R¹, R² und R³ haben hierbei beispielhaft die oben in Gruppe 1 angegebene Bedeutung.

### Gruppe 13

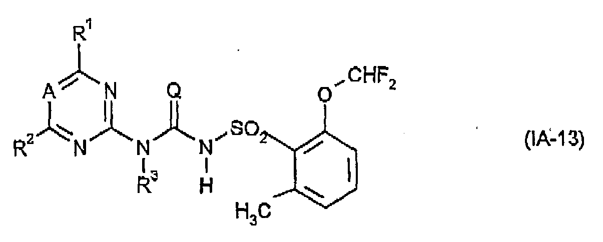

A, Q, R¹, R² und R³ haben hierbei beispielhaft die oben in Gruppe 1 angegebene Bedeutung.

### Gruppe 14

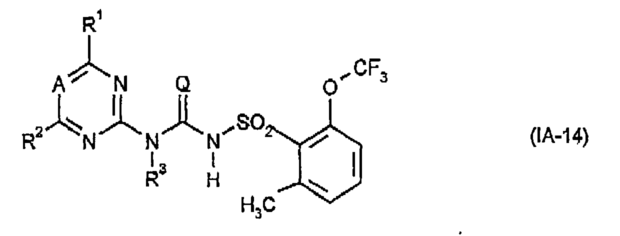

A, Q, R¹, R² und R³ haben hierbei beispielhaft die oben in Gruppe 1 angegebene Bedeutung.

Verwendet man beispielsweise 2-Amino-4-methoxy-6-methyl-pyrimidin und 2- . Ethoxy-6-trifluormethyl-phenylsulfonylisocyanat als Ausgangsstoffe, so kann der Reaktionsablauf beim erfindungsgemäßen Verfahren (a) durch das folgende Formelschema skizziert werden:

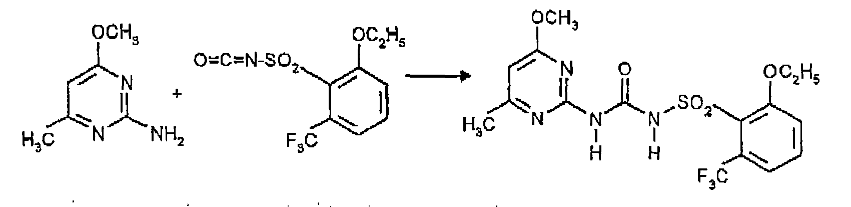

Verwendet man beispielsweise 2-Methoxycarbonylamino-4-methoxy-6-trifluormethyl-1,3,5-triazin und 2-Methyl-6-trifluormethoxy-benzolsulfonamid als Ausgangsstoffe, so kann der Reaktionsablauf beim erfindungsgemäßen Verfahren (b) durch das folgende Formelschema skizziert werden:

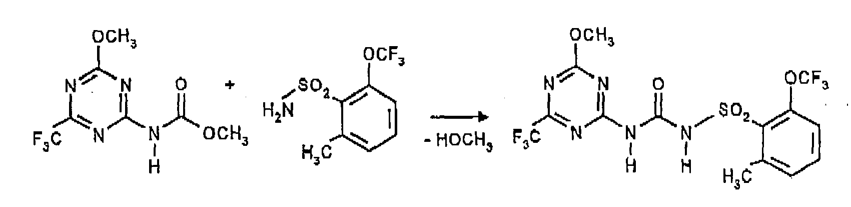

Verwendet man beispielsweise 2-Amino-4-chlor-6-methoxy-pyrimidin und N-(2,6-Dimethoxy-phenylsulfonyl)-O-phenyl-urethan als Ausgangsstoffe, so kann der Reaktionsablauf beim erfindungsgemäßen Verfahren (c) durch das folgende Formelschema skizziert werden;

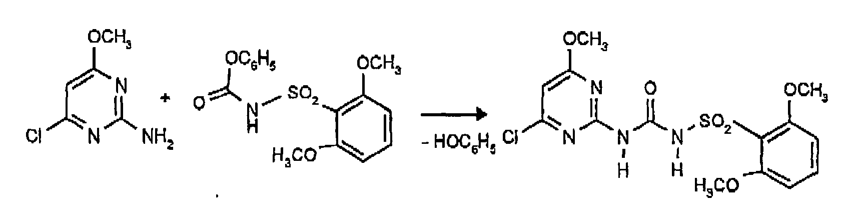

Die bei den erfindungsgemäßen Verfahren (a) und (c) zur Herstellung der Verbindungen der allgemeinen Formel (I) als Ausgangsstoffe zu verwendenden Aminoazine sind durch die Formel (II) allgemein definiert. In der Formel (II) haben A, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, R¹ und R² angegeben wurden.

Die Aminoazine der Formel (II) sind bekannte, zum Teil im Handel erhältliche Synthesechemikalien.

Die beim erfindungsgemäßen Verfahren (a) weiter als Ausgangsstoffe zu verwendenden Arylsulfonyliso(thio)cyanate sind durch die Formel (III) allgemein definiert. In der Formel (III) haben Q, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴ und R⁵ angegeben wurden.

Die Ausgangsstoffe der Formel (III) sind bekannt und/oder können nach an sich bekannten Verfahren hergestellt werden (vgl. DE 3208189, EP 23422, EP 64322, EP 44807, EP 216504, Herstellungsbeispiele).

Man erhält die Arylsulfonyliso(thio)cyanate der Formel (III), wenn man Arensulfonamide der allgemeinen Formel (V) - oben - mit Phosgen bzw. Thiophosgen, gegebenenfalls in Gegenwart eines Alkylisocyanats, wie z.B. Butylisocyanat, gegebenenfalls in Gegenwart eines Reaktionshilfsmittels, wie z.B. Diazabicyclo[2.2.2]-octan, und in Gegenwart eines Verdünnungsmittels, wie z.B. Toluol, Xylol oder Chlorbenzol, bei Temperaturen zwischen 80°C und 150°C umsetzt und nach Ende der Umsetzung die flüchtigen Komponenten unter vermindertem Druck abdestilliert.

Die beim erfindungsgemäßen Verfahren (b) zur Herstellung der Verbindungen der Formel (I) als Ausgangsstoffe zu verwendenden substituierten Aminoazine sind durch die Formel (IV) allgemein definiert. In der Formel (IV) haben A, Q, R¹ und R² vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für A, Q, R¹ bzw. R² angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (IV) sind bekannt und/oder können nach an sich bekannten Verfahren hergestellt werden (vgl. US 4690707, DE 19501174, Herstellungsbeispiele).

Die beim erfindungsgemäßen Verfahren (b) weiter als Ausgangsstoffe zu verwendenden Arensulfonamide sind durch die Formel (V) allgemein definiert. In der Formel (V) haben R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für R⁴, und R⁵ angegeben wurden.

Die Ausgangsstoffe der Formel (V) sind bekannt und/oder können nach an sich bekannten Verfahren hergestellt werden (vgl. DE 3208189, EP 23422, EP 64322, EP 44807, EP 216504, DE 19525162, Herstellungsbeispiele).

Die beim erfindungsgemäßen Verfahren (c) zur Herstellung der Verbindungen der Formel (I) als Ausgangsstoffe zu verwendenden substituierten Arensulfonamide sind durch die Formel (VI) allgemein definiert. In der Formel (VT) haben Q, R⁴ und R⁵ vorzugsweise bzw. insbesondere diejenigen Bedeutungen, die bereits oben im Zusammenhang mit der Beschreibung der Verbindungen der Formel (I) als bevorzugt bzw. als insbesondere bevorzugt für Q, R⁴ bzw. R⁵ angegeben wurden; Z steht vorzugsweise für Fluor, Chlor, Brom, C₁-C₄-Alkoxy oder Phenoxy, insbesondere für Chlor, Methoxy, Ethoxy oder Phenoxy.

Die Ausgangsstoffe der Formel (VI) sind bekannt und/oder können nach an sich bekannten Verfahren hergestellt werden (vgl. die Herstellungsbeispiele).

Als Verdünnungsmittel zur Durchführung der erfindungsgemäßen Verfahren (a), (b) und (c) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Dichlorbenzol, Petrolether, Hexan, Cyclohexan, Dichlormethan, Chloroform, Tetrachlorkohlenstoff; Ether, wie Diethylether, Diisopropylether, Dioxan, Tetrahydrofuran oder Ethylenglykoldimethyl- oder -diethylether; Ketone, wie Aceton, Butanon oder Methyl-isobutyl-keton; Nitrile, wie Acetonitril, Propionitril oder Benzonitril; Amide, wie N,N-Dimethylformamid, N,N-Dimethylacetamid, N-Methyl-formanilid, N-Methyl-pyrrolidon oder Hexamethylphosphorsäutriamid; Ester, wie Essigsäuremethylester oder Essigsäureethylester, Sulfoxide, wie Dimethylsulfoxid; Alkohole, wie Methanol, Ethanol, n- oder i-Propanol, Ethylenglykolmonomethylether, Ethylenglykolmonoethylether, Diethylenglykolmonomethylether, Diethylenglykolmonoethylether, deren Gemische mit Wasser oder reines Wasser.

Die erfindungsgemäßen Verfahren (a), (b) und (c) werden vorzugsweise in Gegenwart eines geeigneten Reaktionshilfsmittels durchgeführt. Als solche kommen alle üblichen anorganischen oder organischen Basen in Frage. Hierzu gehören beispielsweise Erdalkali- oder Alkalimetall- -hydride, -hydroxide, -amide, -alkoholate, -acetate, -carbonate oder -hydrogencarbonate, wie beispielsweise Natriumhydrid, Natriumamid, Natriummethylat, Natriumethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Ammoniumcarbonat sowie tertiäre Amine, wie Trimethylamin, Triethylamin, Tributylamin, N,N-Dimethylanilin, Pyridin, N-Methylpipendin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (a), (b) und (c) in einem größeren Bereich variiert werden. Im allgemeinen arbeitet man bei Temperaturen zwischen -20°C und +150°C, vorzugsweise zwischen 0°C und +120°C.

Die erfindungsgemäßen Verfahren (a), (b) und (c) werden im allgemeinen unter Normaldruck durchgeführt. Es ist jedoch auch möglich, die erfindungsgemäßen Verfahren unter erhöhtem oder vermindertem Druck - im allgemeinen zwischen 0,1 bar und 10 bar - durchzuführen.

Zur Durchführung der erfindungsgemäßen Verfahren (a), (b) und (c) werden die Ausgangsstoffe im allgemeinen in angenähert äquimolaren Mengen eingesetzt. Es ist jedoch auch möglich, eine der Komponenten in einem größeren Überschuß zu verwenden. Die Umsetzung wird im allgemeinen in einem geeigneten Verdünnungsmittel in Gegenwart eines Reaktionshilfsmittels durchgeführt und das Reaktionsgemisch wird im allgemeinen mehrere Stunden bei der erforderlichen Temperatur gerührt. Die Aufarbeitung wird nach üblichen Methoden durchgeführt (vgl. die Herstellungsbeispiele).

Aus den erfindungsgemäßen Verbindungen der allgemeinen Formel (I) können gegebenenfalls Salze hergestellt werden. Man erhält solche Salze in einfacher Weise nach üblichen Salzbildungsmethoden, beispielsweise durch Lösen oder Dispergieren einer Verbindung der Formel (I) in einem geeigneten Lösungsmittel, wie z.B. Methylenchlorid, Aceton, tert-Butyl-methylether oder Toluol, und Zugabe einer geeigneten Base. Die Salze können dann - gegebenenfalls nach längerem Rühren - durch Einengen oder Absaugen isoliert werden.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind. Ob die erfindungsgemäßen Stoffe als totale oder selektive Herbizide wirken, hängt im wesentlichen von der angewendeten Menge ab.

Die erfindungsgemäßen Wirkstoffe können z.B. bei den folgenden Pflanzen verwendet werden:

Dikotyle Unkräuter der Gattungen: Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea, Trifolium, Ranunculus, Taraxacum.

Dikotyle Kulturen der Gattungen: Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen: Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen: Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen, auf Zier- und Sportrasen und Weideflächen und zur selektiven Unkrautbekämpfung in einjährigen Kulturen eingesetzt werden.

Die erfindungsgemäßen Verbindungen der Formel (I) eignen sich insbesondere zur Bekämpfung von monokotylen und dikotylen Unkräutern sowohl im Vorauflaufals auch im Nachauflauf-Verfahren. Sie zeigen starke herbizide Aktivität und ein breites Wirkungsspektrum bei Anwendung auf den Boden und auf oberirdische Pflanzenteile.

Die Wirkstoffe können in die üblichen Formulierungen übergeführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur- und synthetische Stoffe sowie Feinstverkapselungen in polymeren Stoffen.

Diese Formulierungen werden in bekannter Weise hergestellt, z.B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:
z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sagemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z-B. Alkylarylpolyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulvrige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide in Frage, beispielsweise Anilide, wie z.B. Diflufenican und Propanil; Arylcarbonsäuren, wie z.B. Dichlorpicolin-säure, Dicamba und Picloram; Aryloxyalkansauren, wie z.B. 2,4-D, 2,4-DB, 2,4-DP, Fluroxypyr, MCPA, MCPP und Triclopyr; Aryloxy-phenoxy-alkansäureester, wie z.B. Diclofop-methyl, Renoxaprop-ethyl, Fluazifop-butyl, Haloxyfop-methyl und Quizalofop-ethyl; Azinone, wie z.B. Chloridazon und Norflurazon; Carbamate, wie z.B. Chlorpropham, Desmedipham, Phenmedipham und Propham; Chloracetanilide, wie z.B. Alachlor, Acetochlor, Butachlor, Metazachlor, Metolachlor, Pretilachlor und Propachlor; Dinitroaniline, wie z.B. Oryzalin, Pendimethalin und Trifluralin; Diphenylether, wie z.B. Acifluorfen, Bifenox, Fluoroglycofen, Fomesafen, Halosafen, Lactofen und Oxyfluorfen; Harnstoffe, wie z.B. Chlortoluron, Diuron, Fluometuron, Isoproturon, Linuron und Methabenzthiazuron; Hydroxylamine, wie z,B- Alloxydim, Clethodim, Cycloxydim, Sethoxydim und Tralkoxydim; Imidazolinone, wie z.B. Imazethapyr, Imazamethabenz, Imazapyr und Imazaquin; Nitrile, wie z.B. Bromoxynil, Dichlobenil und Ioxynil; Oxyacetamide, wie z.B. Mefenacet; Sulfonylharnstoffe, wie z.B. Amidosulfuron, Bensulfuron-methyl, Chlorimuron-ethyl, Chlorsulfuron, Cinosulfuron, Metsulfuron-methyl, Nicosulfuron, Primisulfuron, Pyrazosulfuron-ethyl, Thifensulfuron-methyl, Triasulfuron und Tribenuron-methyl; Thiolcarbamate, wie z.B. Butylate, Cycloate, Diallate, EPTC, Esprocarb, Molinate, Prosulfocarb, Thiobencarb und Triallate; Triazine, wie z.B. Atrazin, Cyanazin, Simazin, Simetryne, Terbutryne und Terbutylazin; Triazinone, wie z.B. Hexazinon, Metamitron und Metribuzin; Sonstige, wie z.B. Aminotriazol, Benfuresate, Bentazone, Cinmethylin Clomazone, Clopyralid, Difenzoquat, Dithiopyr, Ethofumesate, Fluorochloridone, Glufosinate, Glyphosate, Isoxaben, Pyridate, Quinchlorac, Quinmerac, Sulphosate und Tridiphane.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden. Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 1 g und 10 kg Wirkstoff pro Hektar Bodenfläche, vorzugsweise zwischen 5 g und 5 kg pro ha.

Die Herstellung und die Verwendung der erfindungsgemäßen Wirkstoffe geht aus den nachfolgenden Beispielen hervor.

### Herstellungsbeispiele:

### Beispiel 1

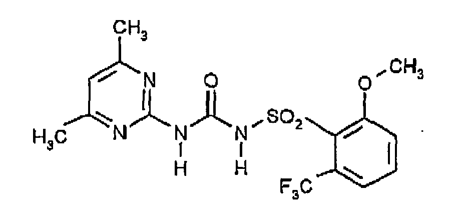

### (Verfahren (b))

Eine Mischung aus 1,43 g (5,6 mMol) 2-Methoxy-6-trifluormethyl-benzolsulfonamid, 1,24 g (5,1 mMol) 2-Phenoxycarbonylamino-4,6-dimethyl-pyrimidin, 0,85 g (5,6 mMol) Diazabicycloundecen (DBU) und 50 ml Acetonitril wird 15 Stunden bei ca. 20°C gerührt. Dann wird im Wasserstrahlvakuum eingeengt und der Rückstand mit 50 ml 1N-Salzsäure und 50 ml Methylenchlorid verrükurt. Die organische Phase wird abgetrennt, mit Wasser gewaschen, mit Natriumsulfat getrocknet und filtriert. Das Filtrat wird im Wasserstrahlvakuum eingeengt, der Rückstand mit i-Propanol digeriert und das kristallin angefallene Produkt durch Absaugen isoliert.

Man erhält 1,6 g (62% der Theorie) N-(4,6-Dimethyl-pyrimidin-2-yl)-N-(2-methoxy-6-trifluormethyl-phenylsulfonyl)-harnstoff vom Schmelzpunkt 190°C.

### Beispiel 2

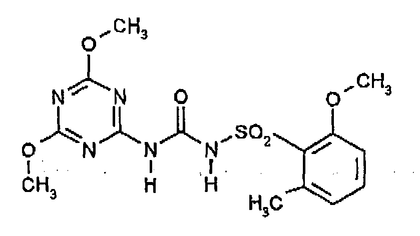

### (Verfahren (b))

Eine Mischung aus 2,0 g (10 mMol) 2-Methoxy-6-methyl-benzolsulfonamid, 4,0 g (10 mMol) N,N-Bis-phenoxycarbonyl-2-amino-4,6-dimethoxy-1,3,5-triazin, 1,1 g (10 mMol) Kalium-t-butanolat und 50 ml Acetonitril wird 15 Stunden bei ca. 20°C gerührt. Dann wird im Wasserstrahlvakuum eingeengt und der Rückstand mit 50 ml 1N-Salzsäure und 50 ml Methylenchlorid verrührt. Die organische Phase wird abgetrennt, mit Wasser gewaschen, mit Natriumsulfat getrocknet und filtriert. Das Filtrat wird im Wasserstrahlvakuum eingeengt, der Rückstand mit i-Propanol digeriert und das kristallin angefallene Produkt durch Absaugen isoliert.

Man erhält 2,1 g (55% der Theorie) N-(4,6-Dimethoxy-1,3,5-triazin-2-yl)-N'-(2-methoxy-6-methyl-phenylsulfonyl)-harnstoff vom Schmelzpunkt 163°C.

### Beispiel 3

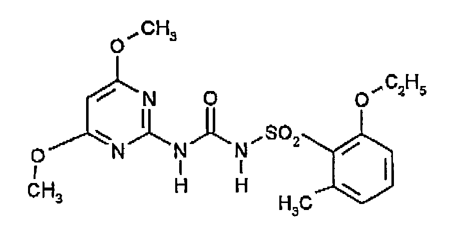

### (Verfahren (c) mit Vorstufe)

Zu einer Mischung aus 2,2 g (10 mMol) 2-Ethoxy-6-methyl-benzolsulfonamid, 2,0 g (20 mMol) Triethylamin und 30 ml Acetonitril werden 1,6 g (10 mMol) Chlorameisensäure-phenylester bei ca. 20°C tropfenweise gegeben und die Mischung wird ca. 30 Minuten bei der angegebenen Temperatur gerührt. Dann gibt man 1,0 g (10 mMol) Methansulfonsäure und 1,6 g (10 mMol) 2-Amino-4,6-di-methoxy-pyrimidin dazu und rührt das Reaktionsgemisch ca. 15 Minuten bei ca. 60°C. Nach Abkühlen auf ca. 20°C wird abgesaugt, das Filtrat im Wasserstrahlvakuum eingeengt und der Rückstand mit 30 ml 1N-Salzsäure vernührt. Nach Absaugen und Trocknen erhält man ein Rohprodukt, das durch Waschen mit Diethylether gereinigt wird.

Ausbeute: 2,2 g (55% der Theorie) N-(4,6-Dimethoxy-pyrimidin-2-yl)-N'-(2-ethoxy-6-methyl-phenylsulfonyl)-harnstoff vom Schmelzpunkt 184°C.

Analog zu den Herstellungsbeispielen 1 bis 3 sowie entsprechend der allgemeinen Beschreibung der erfindungsgemäßen Herstellungsverfahren können beispielsweise auch die in der nachstehenden Tabelle 1 aufgeführten Verbindungen der Formel (I) hergestellt werden.

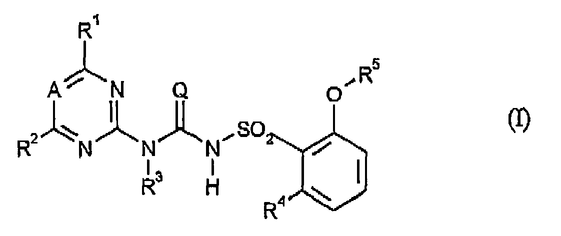

**Tabelle 1: Beispiele für die Verbindungen der Formel (I)**

| Bsp.- Nr. | A | Q | R¹ | R² | R³ | R⁴ | R⁵ | Schmelzpunkt (°C) |
|---|---|---|---|---|---|---|---|---|
| 4 | N | O | CH₃ | OCH₃ | H | CF₃ | C₂H₅ | 115 |
| 5 | N | O | CH₃ | OCH₃ | H | CF₃ | CH₃ | 214 |
| 6 | N | O | OCH₃ | OCH₃ | H | CF₃ | CH₃ | 189 |
| 7 | N | O | OCH₃ | OCH₃ | H | CF₃ | C₂H₅ | 176 |
| 8 | CH | O | CH₃ | CH₃ | H | CF₃ | C₂H₅ | 214 |
| 9 | N | O | CH₃ | OCH₃ | H | CF₃ | C₃H₇-n | 142 |
| 10 | N | O | OCH₃ | OCH₃ | H | CF₃ | C₃H₇-n | 162 |
| 11 | CH | O | CH₃ | CH₃ | H | CF₃ | C₃H₇-n | 212 |
| 12 | N | O | CH₃ | OCH₃ | H | CF₃ | C₃H₇-i | 168 |
| 13 | N | O | OCH₃ | OCH₃ | H | CF₃ | C₃H₇-i | 200 |
| 14 | CH | O | CH₃ | CH₃ | H | CF₃ | C₃H₇-i | 221 |
| 15 | CH | O | Cl | OCH₃ | H | CF₃ | CH₃ | 224 |
| 16 | CH | O | Cl | OCH₃ | H | CF₃ | C₂H₅ | 176 |
| 17 | CH | O | Cl | OCH₃ | H | CF₃ | C₃H₇-n | 169 |
| 18 | N | O | N(CH₃)₂ | OCH₂CF₃ | H | CF₃ | CH₃ | 222 |
| 19 | N | O | N(CH₃)₂ | OCH₂CF₃ | H | CF₃ | C₂H₅ | 209 |
| 20 | N | O | N(CH₃)₂ | OCH₂CF₃ | H | CF₃ | C₃H₇-n | 175 |
| 21 | N | O | N(CH₃)₂ | OCH₂CF₃ | H | CF₃ | C₃H₇-i | 204 |
| 22 | CH | O | OCH₃ | OCH₃ | H | CF₃ | CH₃ | 111 (Zers.) |
| 23 | CH | O | OCH₃ | OCH₃ | H | CF₃ | C₂H₅ | 99 (Zers.) |
| 24 | CH | O | OCH₃ | OCH₃ | H | CF₃ | C₃H₇-n | 180 |
| 25 | CH | O | OCH₃ | OCH₃ | H | CF₃ | C₃H₇-i | 98 (Zers.) |
| 26 | CH | O | CH₃ | CH₃ | H | CH₃ | C₂H₅ | 217 |
| 27 | CH | O | CH₃ | OCH₃ | H | CH₃ | C₂H₅ | 183 |
| 28 | CH | O | H | CH₃ | H | CH₃ | C₂H₅ | 190 |
| 29 | CH | O | CH₃ | CH₃ | H | CH₃ | C₃H₇-n | 194 |
| 30 | CH | O | H | CH₃ | H | CH₃ | C₃H₇-n | 176 |
| 31 | N | O | OCH₃ | OCH₃ | H | CH₃ | C₂H₅ | 181 |
| 32 | N | O | CH₃ | OCH₃ | H | CH₃ | C₂H₅ | 170 |
| 33 | CH | O | CH₃ | OCH₃ | H | CH₃ | C₃H₇-i | 196 |
| 34¹⁾ | CH | O | OCH₃ | OCH₃ | H | CH₃ | 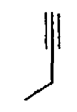 | 204 |
| 35 | CH | O | CH₃ | CH₃ | H | CH₃ | C₃H₇-i | 231 |
| 36 | CH | O | OCH₃ | OCH₃ | H | CH₃ | C₃H₇-i | 190 |
| 37 | CH | O | H | CH₃ | H | CH₃ | C₃H₇-i | 206 |
| 38 | N | O | OCH₃ | OCH₃ | H | CH₃ | C₃H₇-i | 191 |
| 39 | N | O | OCH₃ | OCH₃ | H | CH₃ | C₃H₇-i | 205 |
| 40 | N | O | CH₃ | OCH₃ | H | CH₃ | CH₃ | 193 |
| 41 | CH | O | CH₃ | CH₃ | H | CH₃ | CH₃ | 220 |
| 42 | CH | O | H | CH₃ | H | CH₃ | CH₃ | 206 |
| 43 | CH | O | CH₃ | OCH₃ | H | CH₃ | CH₃ | 172 |
| 44 | CH | O | OCH₃ | OCH₃ | H | CH₃ | CH₃ | 142 |
| 45 | N | O | N(CH₃)₂ | OCH₂CF₃ | H | CH₃ | C₂H₅ | 187 |
| 46 | CH | O | CH₃ | OCH₃ | H | CH₃ | C₃H₇-n | 147 |
| 47 | CH | O | OCH₃ | OCH₃ | H | CH₃ | C₃H₇-n | 164 |
| 48 | N | O | OCH₃ | OCH₃ | H | CH₃ | C₃H₇-n | 156 |
| 49 | N | O | CH₃ | OCH₃ | H | CH₃ | C₃H₇-n | 150 |
| 50 | CH | O | OCH₃ | OCH₃ | H | C₃H₇-i | C₂H₅ | 184 |
| 51 | N | O | OCH₃ | OCH₃ | H | C-₃H₇-i | C₂H₅ | 157 |
| 52 | N | O | N(CH₃)₂ | OCH₂CF₃ | H | CH₃ | CH₃ | 214 |
| 53 | N | O | N(CH₃)₂ | OCH₂CF₃ | H | CH₃ | C₃H₇-i | 202 |
| 54 | CH | O | OCH₃ | OCH₃ | H | CH₃ | 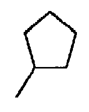 | 171 |
| 55 | N | O | OCH₃ | OCH₃ | H | CH₃ | 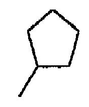 | 198 |
| 56 | N | O | CH₃ | OCH₃ | H | CH₃ | 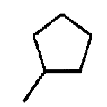 | 183 |
| 57 | N | 0 | OCH₃ | OCH₃ | H | CH₃ | C₄H₉-S | 174 |
| 58 | CH | O | CH₃ | OCH₃ | H | CH₃ | C₄H₉-S | 186 |
| | | | | | | | | |
| 59 | N | O | CH₃ | OCH₃ | H | CH₃ | C₄H₉-S | 177 |
| 60 | CH | O | CH₃ | CH₃ | H | CH₃ | C₄H₉-S | 228 |
| 61 | CH | O | OCH₃ | OCH₃ | H | CH₃ | C₄H₉-S | 207 |
| 62 | CH | O | CH₃ | CF₃ | H | CH₃ | C₃H₇-i | 192 |
| 63 | CH | O | CH₃ | CF₃ | H | CH₃ | CH₃ | 209 |
| 64 | N | O | CH₃ | OCH₃ | H | C₃H₇-i | CH₃ | 136 |
| 65 | CH | O | OCH₃ | OCH₃ | H | CH₃ | CHF₂ | 170 |
| 66 | N | O | CH₃ | OCH₃ | H | CH₃ | CHF₂ | 197 |
| 67 | N | O | OCH₃ | OCH₃ | H | OC₃H₇-n | C₃H₇-n | 140 |
| 68 | CH | O | Cl | OCH₃ | H | OCH₃ | C₃H₇-i | 148 |
| 69 | N | O | CH₃ | OCH₃ | H | OCH₃ | C₃H₇-i | 152 |
| 70 | N | O | CH₃ | OCH₃ | H | OC₄H₉-n | C₄H₉-n | 90 |
| 71 | N | O | CH₃ | OCH₃ | H | OC₃H₇-i | C₃H₇-i | 80 |
| 72 | N | O | CH₃ | OCH₃ | H | OC₂H₅ | C₃H₇-i | 118 |
| 73 | N | O | CH₃ | OCH₃ | Na | OCH₃ | CH₃ | 203 |
| 74 | CH | O | OCH₃ | OCH₃ | H | C₃H₇-i | CH₃ | 164 |
| 75 | CH | O | OCH₃ | OCH₃ | H | C₃H₇-i | C₃H₇-n | 157 |
| 76 | CH | O | CH₃ | CF₃ | H | CH₃ | C₂H₅ | 179 |
| 77 | N | O | CH₃ | CH₃ | H | CF₃ | CH₃ | 155 |
| 78 | N | O | CH₃ | SCH₃ | H | CF₃ | CH₃ | 178 |
| 79 | N | O | CH₃ | N(CH₃)₂ | H | CF₃ | CH₃ | 213 |
| 80 | N | O | CH₃ | OC₂H₅ | H | CF₃ | CH₃ | 121 |
| 81 | N | O | C₂H₅ | OCH₃ | H | CF₃ | CH₃ | 117 |
| 82 | N | O | OCH₂CF₂CHF₂ | N(CH₃)₂ | H | CF₃ | CH₃ | 185 |
| 83 | N | O | CH₃ | OCH₃ | Na | C₃H₇-i | CH₃ | 170 |
| 84 | N | O | OCH₃ | OCH₃ | H | C₃H₇-i | C₃H₇-n | 149 |
| 85 | N | O | OCH₃ | OCH₃ | H | C₃H₇-i | CH₃ | 187 |
| 86 | N | O | CH₃ | OCH₃ | H | C₃H₇-i | C₂H₅ | 163 |
| 87 | CH | O | CH₃ | OCH₃ | H | C₃H₇-i | CH₃ | 175 |
| 88 | CH | O | CH₃ | OCH₃ | H | C₃H₇-i | C₃H₇-i | 152 |
| 89 | CH | O | OCH₃ | OCH₃ | H | C₃H₇-i | C₃H₇-i | 138 |
| 90 | N | O | CH₃ | OCH₃ | Na | CH₃ | CH₃ | 149 |
| 91 | N | O | CH₃ | CH₃ | H | 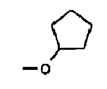 | 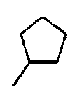 | 125 |
| 92 | N | O | CH₃ | OCH₃ | Na | OC₃H₇-i | C₃H₇-i | 178 |
| 93 | N | O | Cl | OCH₃ | Na | OCH₃ | C₃H₇-i | 172 |
| 94 | N | O | CH₃ | OCH₃ | Na | OC₂H₅ | C₂H₅ | 145 |
| 95²⁾ | N | O | CH₃ | OCH₃ | Na | OCH₃ | C₃H₇-n | NMR-Daten |
| 96 | N | O | OCH₃ | OCH₃ | H | OCH₃ | C₂H₅ | 110 |
| 97 | N | O | OCH₃ | OCH₃ | H | OC₂H₅ | C₂H₅ | 142 |
| 98 | N | O | OCH₃ | OCH₃ | H | OCH₃ | C₃H₇-n | 188 |
| 99 | N | O | OCH₃ | OCH₃ | H | OC₂H₅ | C₄H₉-s | 136 |
| 100 | CH | O | Cl | OCH₃ | H | OCH₃ | C₂H₅ | 146 |
| 101 | CH | O | Cl | OCH₃ | H | OCH₃ | C₃H₇-n | 110 |
| 102 | CH | O | Cl | OCH₃ | H | OCH₃ | C₄H₉-n | 117 |
| 103 | CH | O | Cl | OCH₃ | H | OC₄H₉-n | C₄H₉-n | 135 |
| 104 | CH | O | Cl | OCH₃ | H | OC₂H₅ | C₄H₉-n | 128 |
| 105 | N | O | CH₃ | OCH₃ | H | OC₂H₅ | C₃H₇-n | 110 |
| 106 | N | O | CH₃ | OCH₃ | H | OCH₃ | CH₃ | 177 |
| 107 | N | O | CH₃ | OCH₃ | H | OCH₃ | 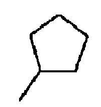 | 166 |
| 108 | N | O | CH₃ | OCH₃ | H | OC₂H₅ | C₄H₉-i | 85 |
| 109 | N | O | CH₃ | OCH₃ | H | OCH₃ | C₂H₅ | 148 |
| 110 | N | O | CH₃ | OCH₃ | H | OCH₃ | C₄H₉-n | 125 |
| 111 | N | O | CH₃ | OCH₃ | H | OC₂H₅ | 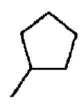 | 130 |
| 112 | CH | O | OCH₃ | OCH₃ | H | CH₃ | CF₃ | 178 |
| 113 | N | O | CH₃ | OCH₃ | H | CH₃ | CF₃ | 178 |
| 114 | CH | O | OCH₃ | OCH₃ | H | C₂H₅ | CF₃ | 147 |
| 115 | N | O | CH₃ | OCH₃ | H | C₂H₅ | CF₃ | 140 |
| 116 | CH | O | OCH₃ | OCH₃ | H | CH₃ | CF₂CHF₂ | 169 |
| 117 | N | O | CH₃ | OCH₃ | H | CH₃ | CF₂CHF₂ | 184 |
| 118 | CH | O | OCH₃ | OCH₃ | H | CH₃ | CH₂CF₃ | 184 |
| 119 | N | O | CH₃ | OCH₃ | H | CH₃ | CH₂CF₃ | 178 |
| 120 | CH | O | OCH₃ | OCH₃ | H | C₂H₅ | CHF₂ | 110 |
| 121 | N | O | CH₃ | OCH₃ | H | C₂H₅ | CHF₂ | 157 |
| 122 | CH | O | OCH₃ | OCH₃ | H | C₃H₇-n | CF₃ | 147 |
| 123 | N | O | CH₃ | OCH₃ | H | C₃H₇-n | CF₃ | 77 |

| | | | | | | | | |
|---|---|---|---|---|---|---|---|---|
| Anmerkungen: ¹⁾ In Beispiel 34 bedeutet 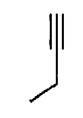 Propargyl = CH₂-C≡CH | | | | | | | | |
| ²⁾ NMR-Daten zu Beispiel 95: ¹H-NMR (300MHz D₂O): δ = 0,96 (t, CH₃); 1,77 (m, O-CH₂-CH₂-CH₃); 2,42 (s, CH₃); 3,87 (s, OCH₃); 3,98 (s, OCH₃); 4,04 (t, O-CH₂-); 6,79 (br.d, 2 aromat H); 7,48 (br.t, 1 aromat H) ppm. | | | | | | | | |

### Ausgangsstoffe der Formel (III):

### Beispiel (III-1)

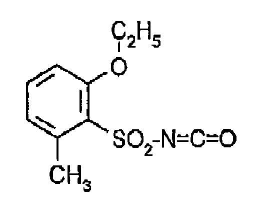

21,5 g (0,1 Mol) 2-Ethoxy-6-methyl-benzolsulfonsäureamid und 10 g (0,1 Mol) n-Butylisocyanat werden in 100 ml Chlorbenzol zum Sieden erhitzt. Bei Rückflußtemperatur wird 4 Stunden Phosgen eingeleitet. Die klare Lösung wird unter vermindertem Druck eingeengt und der Rückstand feindestilliert. Bei einem Druck von 0,8 mbar und einer Kopftemperatur von 135 - 140°C geht 2-Ethoxy-6-methylphenylsulfonylisocyanat über, das in der Vorlage erstarrt.

Man erhält 7,9 g 2-Ethoxy-6-methyl-phenylsulfonylisocyanat als farbloses Produkt vom Schmelzpunkt 40°C.

### Ausgangsstoffe der Formel (IV):

### Beispiel (IV-1)

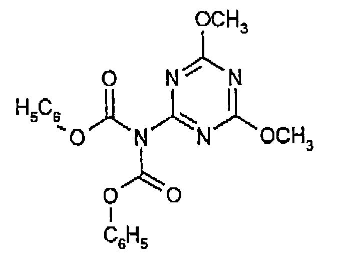

70 g (0,44 Mol) Chlorameisensäure-phenylester werden tropfenweise unter Rühren zu einer Mischung aus 31 g (0,20 Mol) 2-Amino-4,6-dimethoxy-s-triazin und 100 ml Pyridin gegeben. Die Reaktionsmischung wird ca. 15 Stunden bei 20°C bis 25°C gerührt und anschließend im Wasserstrahlvakuum eingeengt. Der Rückstand wird in Wasser aufgenommen und dann mit konz. Salzsäure angesäuert. Das hierbei kristallin angefallene Produkt wird durch Absaugen isoliert.
Man erhält 74,2 g (91% der Theorie) 2-(N,N-Bis-phenoxycarbonyl-amino)-4,6-dimethoxy-s-triazin vom Schmelzpunkt 125°C.

### Ausgangsstoffe der Formel (V):

### Beispiel (V-1)

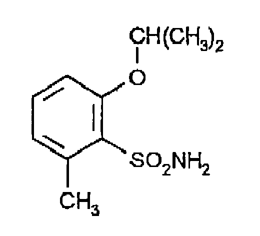

64,6 g (0,26 Mol) 2-Isopropoxy-6-methyl-benzolsulfochlorid werden in 350 ml 25%iger wässriger Ammoniaklösung 12 Stunden bei 20°C gerührt. Das kristallin angefallene Produkt wird anschließend durch Absaugen isoliert.

Man erhält 54 g (90% der Theorie) 2-Isopropoxy-6-methyl-benzolsulfonamid vom Schmelzpunkt 78°C.

### Anwendungsbeispiele:

### Beispiel A

### Pre-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Böden ausgesät. Nach ca. 24 Stunden wird der Boden mit der Wirkstoffzubereitung gespritzt. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit.

Nach drei Wochen wird der Schädigungsgrad der Pflanzen bonitiert in % Schädigung im Vergleich zur Entwicklung der unbehandelten Kontrolle.

Es bedeute:

| | |
|---|---|
| 0 % = | keine Wirkung (wie unbehandelte Kontrolle) |
| 100 % = | totale Vernichtung |

In diesem Test zeigen beispielsweise die Verbindungen gemäß Herstellungsbeispiel 1, 2, 3, 4, 5, 6, 7, 8, 9, 10, 11, 12, 13, 14, 15, 16, 22, 27, 31, 32, 33, 34, 38, 39, 40, 41, 43, 44, 46, 48, 49, 56, 65 und 66 bei Aufwandmengen zwischen 30 g und 125 g a.i. pro Hektar sehr starke Wirkung gegen Unkräuter.

"a.i." = "active ingredient" = Wirkstoff

**Tabelle A: Pre-emergence-Test/Gewächshaus**

| Wirkstoff gemäß Herstellungsbeispiel Nr. | Aufwandmenge (g ai./ha) | Alopecurus | Lolium | Sorghum | Amaranthus | Chenopodium | Steltaria |
|---|---|---|---|---|---|---|---|
| 4 | 60 | 100 | 100 | 100 | 100 | 100 | 95 |
| 5 | 60 | 100 | 100 | 100 | 100 | 100 | 100 |
| 6 | 60 | 100 | 100 | 100 | 100 | 100 | 100 |
| 7 | 60 | 100 | 95 | 100 | 95 | 100 | 95 |
| 1 | 60 | 100 | 100 | 100 | 100 | 100 | 100 |
| 8 | 30 | 100 | 100 | 100 | - | 80 | 90 |
| 9 | 30 | 100 | 100 | 90 | 100 | 100 | 100 |
| 10 | 60 | 100 | 100 | 100 | 100 | 100 | 100 |
| 11 | 60 | 100 | 100 | 100 | 100 | 100 | 95 |
| 12 | 30 | 100 | 100 | 100 | 100 | 95 | 100 |
| 13 | 60 | 100 | 100 | 100 | 100 | 100 | 100 |
| 14 | 30 | 100 | 100 | 95 | - | 95 | 95 |
| 15 | 60 | 100 | 95 | 95 | 95 | 100 | 95 |
| 16 | 30 | 100 | - | 80 | - | 100 | 90 |
| 22 | 30 | 100 | - | 95 | 100 | 100 | 100 |
| 27 | 60 | 100 | 100 | 100 | 100 | 100 | 100 |
| 3 | 60 | 95 | 60 | 80 | 80 | 95 | 95 |
| 31 | 60 | 95 | 100 | 90 | 95 | 100 | 95 |
| 32 | 60 | 95 | 100 | 95 | 100 | 100 | 100 |
| 33 | 30 | 100 | 95 | 100 | - | 70 | 90 |
| 34 | 30 | 100 | - | 90 | - | 90 | 90 |
| 38 | 30 | 100 | 100 | 100 | 80 | 100 | 100 |
| 39 | 30 | 100 | 100 | 100 | 100 | 100 | 100 |
| 40 | 30 | 100 | 100 | 100 | 100 | 100 | 100 |
| 41 | 30 | 95 | 100 | 100 | 60 | 100 | 80 |
| 43 | 30 | 100 | 100 | 100 | 100 | 100 | 100 |
| 44 | 30 | 100 | 100 | 100 | 100 | 100 | 100 |
| 2 | 30 | 100 | 100 | 100 | 100 | 100 | 100 |
| 46 | 60 | 100 | 90 | 100 | 90 | 100 | 80 |
| 48 | 60 | 100 | 100 | 100 | 100 | 100 | 100 |
| 49 | 30 | 100 | 100 | 100 | 100 | 100 | 100 |
| 56 | 60 | 100 | 100 | 90 | 100 | 100 | 100 |
| 65 | 125 | 95 | 70 | - | 95 | 95 | 95 |
| 66 | 60 | 95 | 95 | 95 | 95 | 95 | 95 |

### Beispiel B

### Post-emergence-Test

| | |
|---|---|
| Lösungsmittel: | 5 Gewichtsteile Aceton |
| Emulgator: | 1 Gewichtsteil Alkylarylpolyglykolether |

Zur Herstellung einer zweckmäßigen Wirkstoffzubereitung, vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 - 15 cm haben, so, daß die jeweils gewünschten Wirkstoffmengen pro Flächeneinheit ausgebracht werden. Die Konzentration der Spritzbrühe wird so gewählt, daß in 1000 1 Wasser/ha die jeweils gewünschten Wirkstoffmengen ausgebracht werden.

Nach drei Wochen wird der Schädigungsgrad der Pflanzen bonitiert in % Schädigung im Vergleich zur Entwicklung der unbehandelten Kontrolle.
Es bedeuten:
0 % =keine Wirkung (wie unbehandelte Kontrolle)
100 % =totale Vernichtung

In diesem Test zeigen beispielsweise die Verbindungen gemäß Herstellungsbeispiel 2, 3, 4, 5, 6, 7, 8, 9, 12, 15, 16, 17, 22, 23, 24, 25, 27, 31, 32, 34, 38, 39, 40, 41, 43, 44, 48, 49, 51, 65 und 66 sehr starke Wirkung gegen Unkräuter.

**Tabelle B: Post emergence-Test/Gewächshaus**

| Wirkstoff gemäß Herstellungsbeispiel-Nr. | Aufwandmenge (g ai/ha) | Alopecurus | Echinochloa | Abutilon | Matricaria |
|---|---|---|---|---|---|
| 4 | 30 | 90 | 90 | 100 | 100 |
| 5 | 30 | 80 | 100 | 100 | 100 |
| 6 | 30 | 60 | 100 | 90 | 100 |
| 7 | 30 | 95 | 95 | 95 | 100 |
| 8 | 30 | 80 | 90 | 90 | 100 |
| 9 | 8 | 90 | 80 | 100 | 95 |
| 12 | 30 | 95 | 90 | 95 | 100 |
| 15 | 60 | 70 | 90 | 95 | 100 |
| 16 | 30 | 70 | 70 | 100 | 100 |
| 17 | 30 | - | 70 | 90 | 95 |
| 22 | 30 | 60 | 80 | 95 | 95 |
| 23 | 30 | 60 | 70 | 95 | 95 |
| 24 | 30 | 60 | 70 | 95 | 95 |
| 25 | 30 | 60 | 70 | 95 | 90 |
| 27 | 60 | 95 | 95 | 100 | 90 |
| 3 | 60 | 90 | 80 | 100 | 95 |
| 31 | 60 | 80 | 80 | 100 | 100 |
| 32 | 60 | 70 | 90 | 100 | 100 |
| 34 | 30 | 60 | 80 | 90 | 90 |
| 38 | 30 | 60 | 70 | 95 | 95 |
| 39 | 30 | 90 | 95 | 100 | 100 |
| 40 | 30 | 100 | 95 | 95 | 100 |
| 41 | 30 | 60 | 95 | 90 | 70 |
| 43 | 30 | 60 | 95 | 100 | 100 |
| 44 | 30 | 60 | 90 | 100 | 100 |
| 2 | 30 | 90 | 90 | 95 | 100 |
| 48 | 60 | 95 | 70 | 100 | 90 |
| 49 | 30 | 90 | 80 | 100 | 100 |
| 51 | 60 | 80 | 90 | 100 | 90 |
| 65 | 125 | 90 | 90 | 100 | 100 |
| 66 | 60 | 80 | 80 | 100 | 100 |

## Patentansprüche

1. Substituierte Arylsulfonyl(thio)harnstoffe der allgemeinen Formel (I) 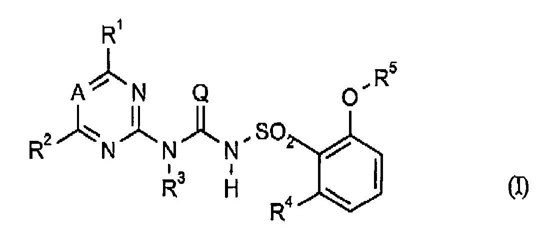 in welcher
A für eine CH-Gruppierung steht,
Q für Sauerstoff oder Schwefel steht,
R¹ für Wasserstoff, Halogen, oder jeweils gegebenenfalls substituiertes Alkyl, Alkoxy, Alkylthio, Alkylamino, Dialkylamino, Cycloalkyl, Cycloalkyloxy oder Heterocyclyloxy steht,
R² für Wasserstoff, Halogen, oder jeweils gegebenenfalls substituiertes Alkyl, Alkoxy, Alkylthio, Alkylamino, Dialkylamino, Cycloalkyl, Cycloalkyloxy oder Heterocyclyloxy steht,
R³ für Wasserstoff oder gegebenenfalls substituiertes Alkyl steht,
R⁴ für jeweils gegebenenfalls substituiertes Alkyl, Alkoxy, Alkenyl, Alkinyl, Alkenyloxy, Alkinyloxy, Cycloalkyl, Cycloalkyloxy oder Cycloalkylalkyl steht, und
R⁵ für Wasserstoff, Formyl, oder jeweils gegebenenfalls substituiertes Alkyl, Alkylcarbonyl, Alkoxycarbonyl, Alkylsulfonyl, Alkenyl, Alkinyl, Cycloalkyl, Cycloalkylcarbonyl, Cycloalkylsulfonyl oder Heterocyclyl steht,
sowie die Salze von Verbindungen der Formel (I),
wobei die Verbindung N-(4,6-Dimethyl-pyrimdin-2-yl)-N'-[2-(1,1,2,2-tetrafluox-ethoxy)-6-methyl-phenysulfonyl]-harnstoff ausgenommen ist.

2. Verbindungen der Formel (I) gemäß Anspruch 1, **dadurch gekennzeichnet, dass** darin
A für eine CH-Gruppierung steht,
Q für Sauerstoff oder Schwefel steht,
R¹ für Wasserstoff, Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Oxetanyloxy, Furyloxy oder Tetrahydrofuryloxy steht,
R² für Wasserstoff oder Halogen, für jeweils gegebenenfalls durch Cyano, Halogen oder C₁-C₄-Alkoxy substituiertes Alkyl, Alkoxy, Alkylthio, Alkylamino oder Dialkylamino mit jeweils 1 bis 4 Kohlenstoffatomen in den Alkylgruppen, für jeweils gegebenenfalls durch Cyano, Halogen, C₁-C₄-Alkyl, oder C₁-C₄-Alkoxy substituiertes Cycloalkyl oder Cycloalkyloxy mit jeweils 3 bis 6 Kohlenstoffatomen, oder für jeweils gegebenenfalls durch Cyano, Halogen, C₁-C₄-Alkyl oder C₁-C₄-Alkoxy substituiertes Oxetanyloxy, Furyloxy oder Tetrahydrofuryloxy steht,
R³ für Wasserstoff oder gegebenenfalls C₁-C₄-Alkoxy, C₁-C₄-Alkylcarbonyl oder C₁-C₄-Alkoxy-carbonyl substituiertes Alkyl mit 1 bis 4 Kohlenstoffatomen steht,
R⁴ für jeweils gegebenenfalls durch Cyano, Halogen oder C₁-C₄-Alkyloxy substituiertes Alkyl oder Alkoxy mit jeweils 1 bis 6 Kohlenstoffatomen in den Alkylgruppen, für jeweils gegebenenfalls durch Halogen substituiertes Alkenyl, Alkinyl, Alkenyloxy oder Alkinyloxy mit jeweils 2 bis 6 Kohlenstoffatomen in den Alkenyl- oder Alkinyl-gruppen, oder für jeweils gegebenenfalls durch Cyano, Halogen oder C₁-C₄-Alkyl substituiertes Cycloalkyl, Cycloalkyloxy oder Cycloalkylalkyl mit jeweils 3 bis 6 Kohlenstoffatomen in den Cycloalkylgruppen und gegebenenfalls 1 bis 4 Kohlenstoffatomen im Alkylteil steht,
R⁵ für Wasserstoff, Formyl, für jeweils gegebenenfalls durch Cyano, Halogen oder C₁-C₄-Alkoxy substituiertes Alkyl, Alkylcarbonyl, Alkoxycarbonyl oder Alkylsulfonyl mit jeweils 1 bis 6 Kohlenstoffatomen in den Alkylgruppen, für jeweils gegebenenfalls durch Halogen substituiertes Alkenyl oder Alkinyl mit jeweils 2 bis 6 Kohlenstoffatomen, für jeweils gegebenenfalls durch Cyano, Halogen oder C₁-C₄-Alkyl substituiertes Cycloalkyl, Cycloalkyl-carbonyl oder Cycloalkylsulfonyl mit jeweils 3 bis 6 Kohlenstoffatomen in den Cycloalkylgruppen, oder für jeweils gegebenenfalls durch Cyano, Halogen, C₁-C₄-Alkyl oder C₁-C₄-Alkoxy substituiertes Oxetanyl, Furyl oder Tetrahydrofuryl steht,
wobei die Verbindung N-(4,6-Dimethyl-pyrimidin-2-yl)-N'-[2-(1,1,2,2-tetra-fluorethoxy)-6-methyl-phenylsulfonyl]-harnstoff ausgenommen ist,
sowie die Natrium-, Kalium-, Magnesium-, Calcium-, Ammonium-, C₁-C₄-Alkylammonium-, Di-(C₁-C₄-alkyl)-ammonium-, Tri-(C₁-C₄-alkyl)-ammonium-, Tetra-(C₁₋C₄-alkyl)-ammonium-, Tri-(C₁-C₄-alkyl)-sulfonium-, C₅- oder C₆-Cycloalkyl-ammonium- und Di (C₁-C₂-alkyl)-benzyl-ammonium-Salze von Verbindungen der Formel (I).

3. Verbindungen der Formel (I) gemäß Anspruch 1, **dadurch gekennzeichnet, dass** darin
A für eine CH-Gruppierung steht,
Q für Sauerstoff oder Schwefel steht,
R¹ für Wasserstoff, Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n- oder i-Propylthio, Methylamino, Ethylamino, n- oder i-Propylamino, Dimethylamino oder Diethylamino steht,
R² für Fluor, Chlor, Brom oder jeweils gegebenenfalls durch Cyano, Fluor, Chlor, Methoxy oder Ethoxy substituiertes Methyl, Ethyl, n- oder i-Propyl, Methoxy, Ethoxy, n- oder i-Propoxy, Methylthio, Ethylthio, n-oder i-Propylthio, Methylamino, Ethylamino, n- oder i-Propylamino, Dimethylamino oder Diethylamino steht,
R³ für Wasserstoff oder gegebenenfalls durch Methoxy, Ethoxy, n- oder i-Propoxy, Acetyl, Proionyl, n- oder i-Butyroyl, Methoxycarbonyl, Ethoxycarbonyl, n- oder i-Propoxycarbonyl substituiertes Methyl oder Ethyl steht,
R⁴ für jeweils gegebenenfalls durch Cyano, Fluor, Chlor, Methoxy oder Ethoxy substituiertes Methyl, Ethyl, n- oder i-Propyl, n-, i-, s- oder t-Butyl, Methoxy, Ethoxy, n- oder i-Propoxy, n-, i-, s- oder t-Butoxy, für jeweils gegebenenfalls durch Fluor, Chlor oder Brom substituiertes Propenyl, Butenyl, Propinyl, Butinyl, Propenyloxy, Butenyloxy, Propinyloxy oder Butinyloxy, oder für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropyloxy, Cyclobutyloxy, Cyclopentyloxy, Cyclohexyloxy, Cyclopropylmethyl, Cyclobutylmethyl, Cyclopentylmethyl oder Cyclohexylmethyl steht,
R⁵ für Wasserstoff, Formyl, für jeweils gegebenenfalls durch Cyano, Fluor, Chlor, Methoxy, Ethoxy, n- oder i-Propoxy substituiertes Methyl, Ethyl, n- oder i-Propyl, n-, i-, s- oder t-Butyl, Acetyl, Propionyl, n- oder i-Butyroyl, Methoxycarbonyl, Ethoxycarbonyl, n- oder i-Propoxycarbonyl, Methylsulfonyl, Ethylsulfonyl, n- oder i-Propylsulfonyl, für jeweils gegebenenfalls durch Fluor, Chlor oder Brom substituiertes Propenyl, Butenyl, Propinyl oder Butinyl, oder für jeweils gegebenenfalls durch Cyano, Fluor, Chlor, Brom, Methyl, Ethyl, n- oder i-Propyl substituiertes Cyclopropyl, Cyclobutyl, Cyclopentyl, Cyclohexyl, Cyclopropylcarbonyl, Cyclobutylcarbonyl, Cyclopentylcarbonyl, Cyclohexylcarbonyl, Cyclopropylsulfonyl, Cyclobutylsulfonyl, Cyclopentylsulfonyl oder Cyclohexylsulfonyl steht,
wobei die Verbindung N-(4,6-Dimethyl-pyrimidin-2-yl)-N'-[2-(1,1,2,2-tetra-fluorethoxy)-6-methyl-phenylsulfonyl]-harnstoff ausgenommen ist.

4. Verfahren zur Herstellung von Verbindungen der Formel (I) gemäß Anspruch 1, **dadurch gekennzeichnet, dass** man
(a) Aminoazine der allgemeinen Formel (II) 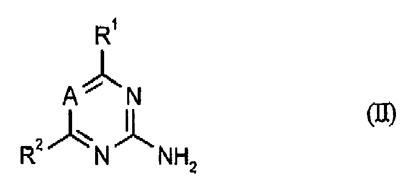 in welcher
A, R¹ und R² die in Anspruch 1 angegebene Bedeutung haben,
mit Arylsulfonyliso(thio)cyanaten der allgemeinen Formel (III) 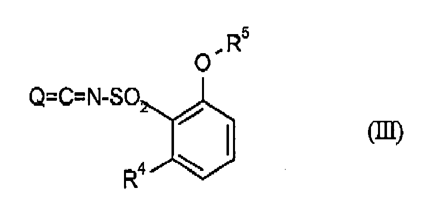 in welcher
Q, R⁴ und R⁵ die in Anspruch 1 angegebene Bedeutung haben,
gegebenenfalls in Gegenwart eines Reaktionshilfsmittels und gegebenenfalls in Gegenwart eines Verdünnungsmittels umsetzt,
oder dass man
(b) substituierte Aminoazine der allgemeinen Formel (IV) 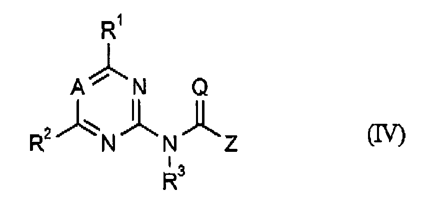 in welcher
A, Q, R¹ und R² die oben angegebene Bedeutung haben,
Z für Halogen, Alkoxy oder Aryloxy steht und
R³ die in Anspruch 1 angegebene Bedeutung hat oder für die Gruppierung - C(Q)-Z steht,
mit Arensulfonamiden der allgemeinen Formel (V) 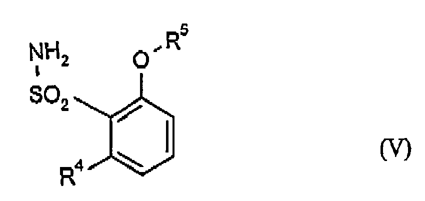 in welcher
R⁴ und R⁵ die oben angegebene Bedeutung haben,
gegebenenfalls in Gegenwart eines Reaktionshilfsmittels und gegebenenfalls in Gegenwart eines Verdünnungsmittels umsetzt,
oder dass man
(c) Aminoazine der allgemeinen Formel (II) 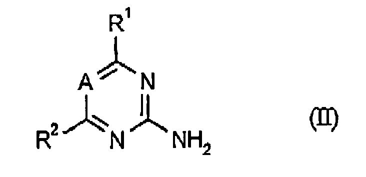 in welcher
A, R¹ und R² die oben angegebene Bedeutung haben,
mit substituierten Arensulfonamiden der allgemeinen Formel (VI) 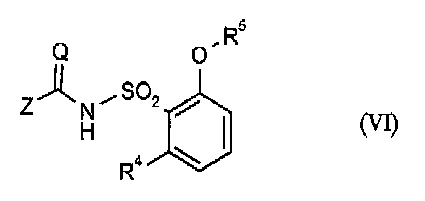 in welcher
Q, R⁴und R⁵ die oben angegebene Bedeutung haben und
Z für Halogen, Alkoxy oder Aryloxy steht,
gegebenenfalls in Gegenwart eines Reaktionshilfsmittels und gegebenenfalls in Gegenwart eines Verdünnungsmittels umsetzt,
und gegebenenfalls die nach den Verfahren (a), (b) und (c) erhaltenen Verbindungen der Formel (I) nach üblichen Methoden in Salze überführt.

5. Herbizide Mittel, **gekennzeichnet durch** einen Gehalt an mindestens einer Verbindung der Formel (I) gemäß Anspruch 1.

6. Verwendung von Verbindungen der allgemeinen Formel (I) gemäß Anspruch 1 zur Bekämpfung von unerwünschtem Pflanzenwachstum.

7. Verfahren zur Bekämpfung von Unkräutern, **dadurch gekennzeichnet, dass** man Verbindungen der allgemeinen Formel (I) gemäß Anspruch 1 auf die Unkräuter oder ihren Lebensraum einwirken lässt.

8. Verfahren zur Herstellung von herbiziden Mitteln, **dadurch gekennzeichnet, dass** man Verbindungen der allgemeinen Formel (I) gemäß Anspruch 1 mit Streckmitteln und/oder oberflächenaktiven Mitteln vermischt.
